# EUROPEAN PATENT SPECIFICATION

(11) **EP 0 567 321 B1**
(45) Date of publication and mention of the grant of the patent: **15.07.1998**
(21) Application number: 93303112.2
(22) Date of filing: 21.04.1993
(51) Int. Cl.: A61M 25/06, A61M 25/01

(54) **Intravenous catheter with needle guard**
Intravenöser Katheter mit Nadelschutzvorrichtung
Cathéter intraveineux avec protecteur d'aiguille

(30) Priority: 21.04.1992 US 871883
(43) Date of publication of application: 27.10.1993
(73) Proprietor: CRITIKON, INC., Tampa Florida 33634 (US)
(72) Inventor: Chang, Joseph J., Tampa, Florida 33625 (US)
(74) Representative: Fisher, Adrian John

(56) References cited:
- EP-A- 0 033 207
- EP-A- 0 451 040
- EP-A- 0 459 954
- WO-A-89/00865
- US-A- 5 000 740

## Description

### FIELD OF THE INVENTION

The present invention relates to an intravenous catheter with needle guard. More specifically, the invention relates to a catheter assembly which allows the retraction of the needle within a protective guard as soon as the catheter tube is placed into a blood vessel, thus protecting the user from accidental needle injury.

### BACKGROUND OF THE INVENTION

The conventional one-way intravenous catheter originates from the need of infusing pharmaceutical liquids (flebochlysis) or blood (transfusion) into the venous system, and of drawing blood therefrom (donation).

An intravenous catheter assembly typically comprises a thin tube usually made of a material such as Teflon (Registered Trade Mark), of a size which depends on the required use, attached to a plastic hub, and provided with standard connecting means for connection to the infusion or blood drawing sets. The catheter tube is inserted into the vein of a patient by means of an inner coaxial stainless steel introducer needle, which is withdrawn by retracting it from the catheter tube immediately after insertion of the catheter into the patient's vein. The needle is attached to a separate plastic support.

Catheters are generally employed in hospitals for administering pharmaceutical solutions directly into the venous system. The port contained on the catheter is connected to an infusion set, which is in its turn connected to the container of the liquid to be infused. After the catheter has been safely placed into the patient's vessel, and once the needle is withdrawn from the catheter, the user should dispose of the needle, but very often he first connect the infusion set to the catheter port, and then tapes the catheter to the patient's skin. Sometimes the user simply drops the needle nearby, so that he can retrieve it and dispose of it later.

The various handling of the needle, once it has been removed, bring about considerable risk of accidental needle injury, which may have serious consequences if the needle is infected by the patient's blood. This problem is particularly critical in the hospital departments where serious infectious diseases such as hepatitis or AIDS are treated.

In order to reduce the danger coming from used needles, there have been provided plastic sheaths or caps that should be employed to cover the needle right after use, but this operation may easily cause accidental injury right when the sheath is being placed again on the needle.

A device directed to the solution of the above-mentioned problem is disclosed in Patent No. 5,000,740. The one-way catheter disclosed in the above-mentioned patent comprises a catheter tube with the relevant hub, a needle located within the tube and attached to a specially designed plastic housing, a protective needle guard provided with a longitudinal slot which may slide with respect to the housing so as to completely cover the needle, and a removable protective sheath which surrounds the needle and catheter tube prior to use. As initially assembled, the needle guard is completely retracted with respect to the needle housing, and the needle and catheter tube are covered by the above mentioned sheath, which is releasably connected by means of a flange to said needle guard. Prior to use, the sheath is removed and the catheter is inserted into the patient's vessel, as with typical non-shielded catheter usage. Once the tip of the needle has reached the blood vessel, the user, while holding the device by the needle housing, advances the tubular needle guard, thereby disengaging the catheter hub from the needle and causing the retraction of the needle within the needle guard starting from the rear end of the needle. Thus, the immediate protection of the needle after use is obtained without requiring the user's hands to pass close to the needle tip, and therefore allows a quite safe operation.

The disclosed device in Patent No. 5,000,740, while affording the required safety, has, however, a rather expensive to manufacture. In particular two additional elements are required, i.e. the protective sheath and the tubular needle guard, whose construction is particularly complicated.

EP-A-459954, which is used as a basis for the preamble of claim 1, discloses a catheter assembly provided with a needle guard slidable with respect to a needle housing so as to completely cover the needle both before and after use.

The object of the present invention is to provide a one-way intravenous catheter which allows the same safety and ease of operation as other safety-shielded catheters, while being of a simple construction and involving low production costs. Such simple construction results in contribution to the safety against blood borne diseases by becoming widely available to the hospital market.

### SUMMARY OF THE INVENTION

According to the present invention, a catheter assembly and a catheter are provided as defined in the claims. The catheter assembly discloses a side-ported stickless catheter with a plug to minimize blood contact and can be described as follows:

The stickless feature of the device is similar to above-mentioned designs with following changes: a) The orientation of the needle guard is reversed. This positions the locking track at the bottom of the guard, and allows formation of a primary push-off tab and two or three secondary tabs at the top for ease of threading the catheter into the patient; b) A similar needle housing is now provided with a blunt nose section; c) A plug made of elastic material to be placed into the catheter hub and serves a barrier to keep blood contained in the hub. The plug has an oversized liner section and a flange section which has a diameter no less than the hub's outer diameter at its proximal end.

When fully assembled, the needle is pierced through the flange section of the plug. The liner section of the plug is completely inserted into the hub while the flange is resting at the proximal end of the hub. The fixed thickness flange serves as a positive stop for the flat nose tip of the needle housing. Therefore, only a fixed thickness of the elastic material is present between the hub base and the nose flat. Upon insertion and hooding, the blood will start to backflow. This proposed design allows the plug to create a continuous wiping action on the cannula to contain the blood in the hub, and to serve as a balancing means to facilitate threading of the catheter. The plug stays with the hub throughout the entire procedure until after side port liner connection is to be made.

There is proposed to supply a conventional catheter with a housing element for the needle, substantially in the form of a hollow cylinder, with a peripheral finger grip coaxial to said cylinder and connected to it by means of a longitudinal connecting element, and with one simple tubular needle guard longitudinally cut by a guiding slot, slidable on said longitudinal connecting element of the needle housing, so as to completely surround all the elements of the catheter but the above-mentioned peripheral finger grip. The shape of the said longitudinal connecting element and that of the needle guard slot which cooperates with the former are such as to have an irreversibly locked position with the needle completely retracted within the needle guard.

Since the needle guard has a tubular shape, open at least at the front end, it may slide with respect to the needle housing, and therefore with respect to the whole catheter, between two extreme positions, the first one of complete retraction, with the needle and catheter tube fully uncovered (use position) and the other of complete extension, with the needle (and possibly the catheter tube) completely covered by the needle guard.

The catheter does not require the use of a further element such as a sheath, to be placed on the needle in the initial assembly, as the needle is already protected by the tubular needle guard in its fully extended position. Prior to use, the needle guard is pushed backwards, thus uncovering the needle and the catheter tube, and after that the catheter has been placed into the blood vessel, the needle guard is returned to its extended position, thus protecting the needle. At the end of the stroke the locking mechanism of the needle guard locks the needle in the needle housing thereby avoiding any accidental needle injury.

Preferably, the central portion of the needle housing is provided with two transverse guiding ribs whose outer diameter is sized to fit the internal surface of the tubular needle guard. As it will be clear with reference to the enclosed drawings, the function of these two transverse ribs is to smoothly guide the sliding of the needle within the needle guard while keeping the needle perfectly coaxial with the needle guard. In an alternative embodiment of the invention, there are provided four longitudinal guiding ribs, projecting from the central cylindrical portion of the needle housing and uniformly spaced around it. The height of these longitudinal ribs are controlled such that the central cylindrical portion of the housing fits the internal surface of the tubular needle guard.

The peripheral finger gripping means of the needle housing preferably has a partially interrupted cylindrical internal surface, and a substantially parallelepiped outer surface with two contoured opposite faces.

The longitudinal connecting element which connects the central body of the needle housing to the peripheral finger gripping means, and which cooperates with the longitudinal slot for guiding said needle guard, is preferably made of two separate portions, both tapered to effect a greater width towards the central portion of this connecting element, and separated by an aperture in their central portion. In this embodiment the locking means provided on the connecting element are represented by an aperture in the central portion of the connecting element, while the corresponding locking means on the needle guard are represented by two opposed teeth projecting long the guiding slot. These teeth are configured so as to irreversibly engage with said aperture in the central portion of the connecting element to lock the needle within the needle guard.

Preferably, the longitudinal slot is provided with a second pair of opposed teeth having a rounded profile and located towards the front end of the needle guard with respect to the first pair of opposed teeth. As it will be clear with reference to the enclosed drawings, the second pair of teeth provides a non-irreversible retainment mechanism on which the catheter assembly is positioned before use, in order to prevent accidental sliding of the needle and catheter tube from the needle guard before use.

The catheter assembly may also comprise a tab projecting upwards from the front end of the tubular needle guard, which may be held by the user when retracting or advancing the needle guard with respect to the needle housing.

The central cylindrical portion of said needle housing is preferably made of a transparent or translucent material, so that the cylindrical central portion may be used as a flash chamber allowing the flow of blood to be apparent as soon as the needle reaches a blood vessel. The flash chamber may be closed by a microporous plug.

The structure of the catheter assemblies according to this invention, as well as the relevant use and advantages, will be made clear with reference to some preferred embodiments thereof, which are shown in the enclosed drawings, wherein:

### DESCRIPTION OF THE DRAWINGS

Figure 1 is a top view of a first embodiment of a known catheter assembly with needle guard;
Figure 2 is a top view of the needle guard of the catheter assembly of Figure 1;
Figure 3 is a top view of the needle housing of the catheter assembly of Figure 1;
Figure 4 is a bottom view of the same catheter assembly of Figure 1 shown without the catheter tube in place;
Figure 5 is a top view of a second embodiment of a known needle guard of the catheter assembly;
Figure 6 is a side view of the needle guard of Figure 5 taken from the right-hand side;
Figure 7 is a top view of the central portion of a second embodiment of the needle housing;
Figure 8 is a cross-sectional view of the needle housing of Figure 7, taken along the line 8-8;
Figure 9 is a perspective assembly view of an embodiment of the catheter assembly of this invention without a needle housing;
Figure 10 is a perspective view of the needle housing used in conjunction with the assembly of Figure 9; and
Figure 11 is a cross-sectional view of the assembled needle housing and catheter with an elastic plug in place.

### DETAILED DESCRIPTION OF THE INVENTION

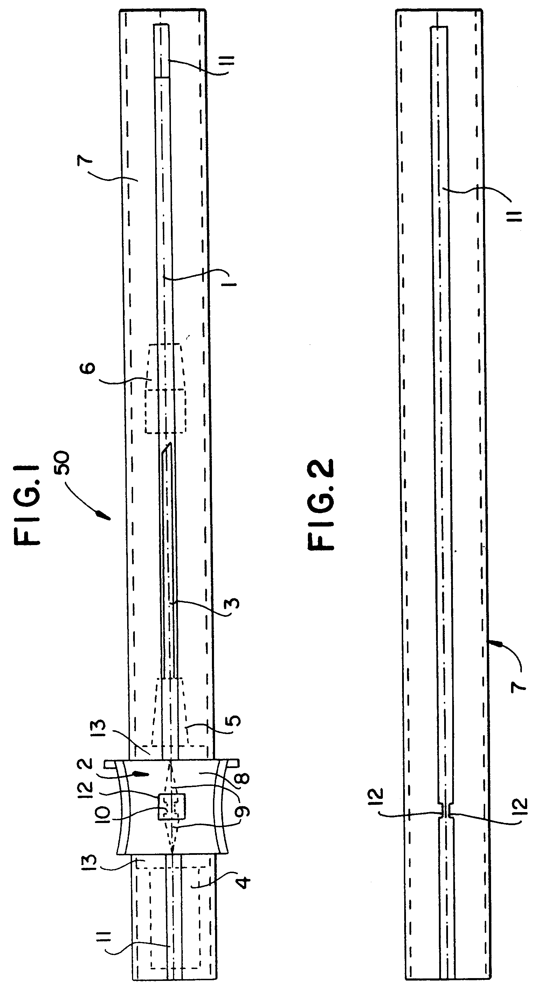

Figure 1 shows a known catheter assembly 50 with most of its elements and with the catheter tube 1 shown separate for a better understanding.

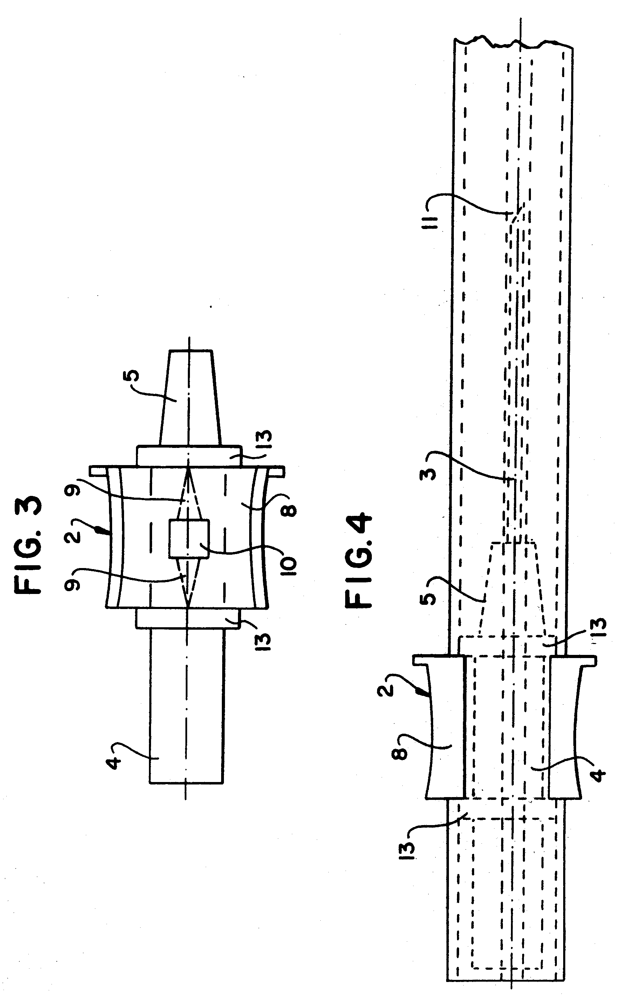

The housing 2 of the needle 3 (shown in detail in Figure 3) comprises a substantially cylindrical central body 4, which is attached to the needle 3. The front portion 5 of the needle housing 2 is slightly tapered (male luer ISO) so as to perfectly engage the tapered internal surface of the catheter hub 6 (female liner ISO).

The central body 4 of the needle housing 2 is made of a transparent or translucent material, so as to be used as a flash chamber, and is generally closed by a microporous plug (not shown) as is well known in the art. The microporous plug allows the passage of gases while preventing the liquid flow out of the flash chamber and is made of, for instance, non-toxic porous polyethylene. The central body 4 may coaxially slide within a needle guard 7. The needle guard 7 is made of transparent plastic material.

The central body 4 is surrounded by a peripheral finger gripping element 8 coaxial with the catheter, which has an inner interrupted cylindrical surface sized to fit around the tubular needle guard 7, and a substantially parallelepiped shaped outer surface with two contoured opposite sides, so as to be easily gripped by the user with the index and the thumb. The gripping element 8 is the only element of the catheter assembly which is not contained within the needle guard 7, but extends around it and can almost completely surround it. In particular, the gripping element 8 is seen as an integral element in the front views of Figures 1 and 3, while in the opposite view of Figure 4 only two separate sections thereof are visible.

The finger gripping element 8 is connected to the central body 4 of the needle housing 2 by means of a longitudinal connecting element divided into two separate sections 9 shown by broken lines in Figures 1 and 3. The cross-section of both sections 9 is tapered towards the central portion of the connecting element, from a smaller to a greater width, and the central portion is interrupted by a square aperture 10.

The needle guard 7, shown in detail in Figure 2, is supplied with a longitudinal guiding slot 11, which engages with the two sections 9 of the longitudinal connecting element. The slot 11 has two opposed teeth 12 which are part of an irreversible locking mechanism, together with the aperture 10 on the needle housing 2. Figure 1 shows the needle housing 2 in the locked position, with the teeth 12 engaged in the aperture 10. The tapered cross-section of the sections 9 of the connecting element is designed to guide the two teeth 12 apart from each other while the needle housing 2 slides with respect to the needle guard 7, until the locking position is reached.

When advancing the needle 3 and catheter 6 and 1 prior to use, the longitudinal slot 11 cooperates with the two sections 9 of the connecting element in guiding the needle housing 2 out of the needle guard 7. This causes the needle 3 and catheter 1 to be exposed for insertion into the body. After the catheter tube 1 has been placed into the patient's vein, the same guiding system helps the retraction of the needle 3 within the needle guard 7. When the needle housing 2 is pushed towards a position fully retracted with respect to the needle guard 7, the two teeth 12 on the slot 11 cooperate with the sections of the connecting element and serves to irreversibly lock the catheter assembly in the position shown in Figure 1. The latter position is reached after use, when the catheter assembly, deprived of the catheter tube 1 and hub 6, is going to be disposed of.

In order to obtain concentricity of the needle housing 2 with the needle guard 7, two transverse guiding ribs 13 are provided around the central body 4 of the needle housing 2. The outer diameter of the guiding ribs 13 is such that they perfectly fit within the tubular needle guard 7.

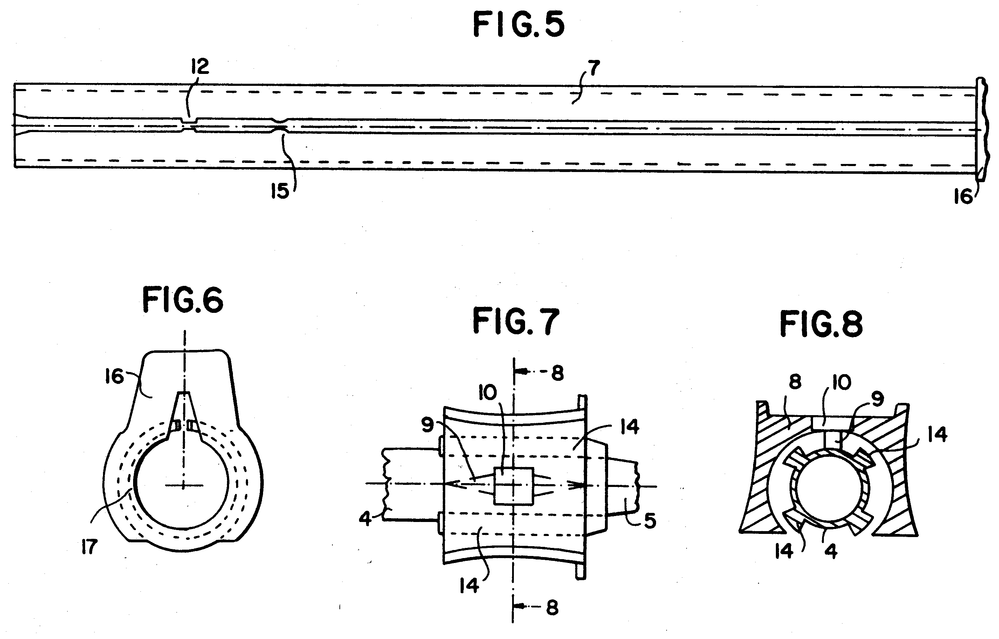

A different embodiment of the needle housing 2 is shown if Figures 7 and 8, where corresponding elements are shown by the same numeral as in the previous figures. Here, however, there are described four longitudinal ribs 14. The four longitudinal guiding ribs 14 have the same function as the two transverse guiding ribs 13 of Figures 1, 3 and 4.

A second embodiment of the needle guard is shown if Figures 5 and 6, where corresponding elements are shown by the same numerals as in the previous figures. In this embodiment, a pre-lock formed by the teeth 15 has been added, in order to provide a stable position in which the catheter assembly can be stored before use, thereby avoiding that the needle 3 accidentally slides out of the housing 7 e.g. during transport. Since the pre-lock position must not be irreversible, the two teeth 15 have a rounded profile.

A further feature of the embodiment shown in Figures 5 and 6 is the push tab 16, projecting upwards from the front end of the needle guard 7. The tab 16 may be gripped while making the needle guard 7 slide with respect to the needle housing 2.

As shown in Figure 6, the inner diameter of the collar 17 of the tab 16 is slightly smaller than inner diameter of the needle guard 7. When the catheter hub 6, mounted on the needle 3, is pushed forward for use, it is made to pass the narrowed section of the collar 17 by means of a slight pressure. As a result, the catheter hub 6 and tube 1 are prevented from being retracted together with the needle 3 after that the catheter tube has reached the blood vessel, as the catheter hub 6 abuts on the collar 17. Thereafter, the needle 3 is retracted. Catheter 1 remains in the patient, and can be removed from needle guard 7 by gently twisting needle guard 7 so that hub 6 is urged around collar 7, so as to separate the two mechanisms. Of course, needle 3 has by this time been safely locked into guard 7 by engaging aperture 10 with teeth 12.

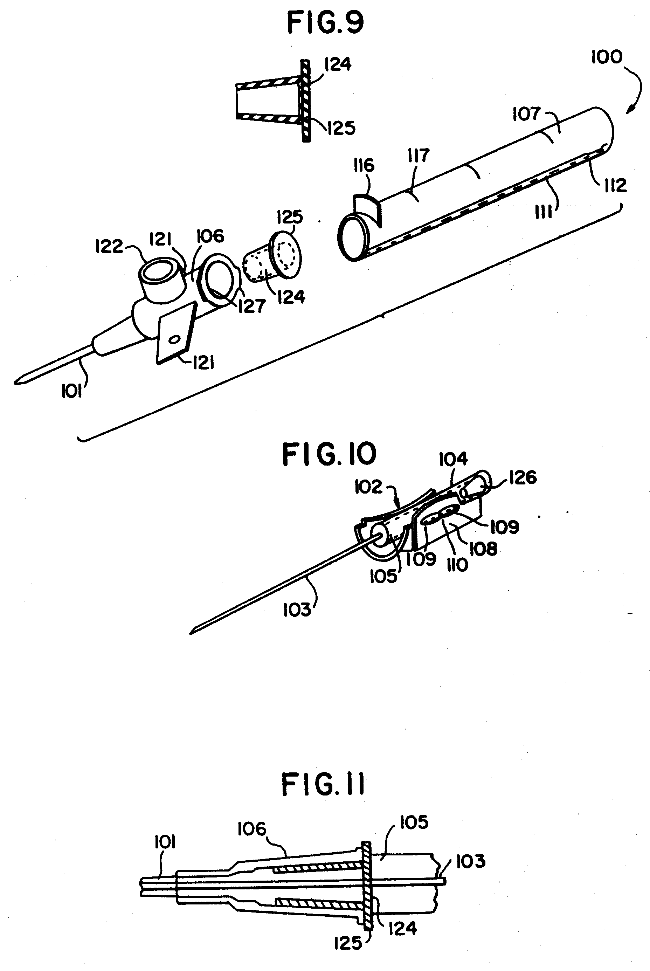

As described in Figures 9, 10 and 11, there is disclosed an embodiment of a catheter assembly 100 in accordance with the present invention. This catheter assembly contains a catheter 101 attached to a hub 106. It can be inserted within a needle guard 107 similar to the previous embodiments. Also, a needle 103 fits within the catheter 101. This needle 103 is attached to housing 102. Housing 102 has grips 108 which fit around needle guard 107. Also, housing 102 now has a clear center 109 with a flash plug 126 attached to it. Further, housing 102 has guides 109 and aperture 110 for attachment to slide 111 contained in needle guard 107. Furthermore, there is contained on needle guard housing a flat nose 105 which will be further described herein.

The catheter 101 attached to hub 106 is a side port catheter with side port feeding member 122 and a pair of taping wings 121. The needle guard 107 contains locking means 112 similar to locking means 12 on previous embodiments. There is also finger tab 116 on needle guard 107.

Importantly, there is described herein an elastic plug 124. This plug contains flanges 125. As seen in Figure 11, the plug 124 fits capable within the rear of catheter hub 106. Ultimately, needle 103 will be inserted within plug 124 and then into catheter hub 106 so that it can be threaded through catheter 101. The needle 103, when so attached, can be inserted as far as nose 105 on housing 102 allows it to. In this way, the abutting of nose 105 on plug 124 serves as a positive stop, and therefore tolerancing is made more readily easy.

As described in the Summary of the Invention, the side port catheter of this invention contains the following valuable features. First, it will be noticed that the needle guard 107 is now in an opposite orientation. This places the track 111 at the patient facing side of the needle guard 107, and allows for positioning of the push-off tab 116 and two or three secondary tabs at the top of the needle guard 107 for threading. Also, the nose section 105 of the housing provides for a positive stop, as previously described. Further, the plug 124, made of elastic material is placed between the catheter hub 106 and serves as a barrier to keep blood contained within the catheter hub 106. The oversized flange 125 of the plug 124 (which is no less than the diameter of the catheter hub 106) allows for easy insertion and engagement with the catheter hub 106.

When fully assembled, the needle 103 pierces the plug 124. The luer section of the plug 124 is completely inserted into the catheter hub, while the flange 125 is abutting the proximal end of the catheter hub 106. The thickness of the flange 125 serves as a positive stop for the flat-nosed tip of the needle housing 102. Therefore, only a fixed thickness of elastic material for the flange 125 is present between the hub 106 and the needle housing nose 105. This allows for easier length control of the catheter 101.

In use, this embodiment of catheter assembly 100 is used much as the catheter assembly 50. Upon insertion, the blood will start to backflow into the transparent area 104 of the needle housing 102 to provide flash. However, unlike the first embodiment, this design allows the plug to 1) create a continuous wiping action between the needle, and the catheter hub 106, and 2) serve to facilitate threading of the needle 103 into the catheter 101. The plug 124 stays with the hub 106 throughout the entire procedure until luer connection is desired to be made, either at the side port 122 or at the proximal end of the catheter hub which abuts the plug 124. There, the plug is removed and luer connection is made on luer lugs 127.

Of course, in use of this catheter assembly 100, the needle 103 continues to be locked by causing the aperture 110 to engage with teeth 112. Therefore, the protective nature of this invention continues to be true.

It is evident from the foregoing that the catheter assembly according to the invention affords the highest safety against accidental needle injury, as it prevents the needle tip from coming into contact with the user's hands either before use or after. In particular, no additional sheath is to be removed before use, and the used needle is retracted within the needle guard as it is extracted from the patient's blood vessel.

Furthermore, the construction of the catheter assembly according to the invention is particularly simple, and the relevant production costs are reasonably low.

## Claims

1. A catheter assembly (100) comprising:
a catheter (101) attached to a catheter hub (106);
a needle (103) capable of heing inserted into said catheter hub (106) and into said catheter (101);
a needle housing (102) having a distal end for holding said needle (103), a cylindrical central body to which said needle (103) is attached, gripping means (108) for moving said needle housing (102), and longitudinal guide means (109) connecting said gripping means (108) to said central body;
a cylindrical needle guard (107) with a pair of open ends, said guard (107) having a guide capable of mating with said guide means (109) of said needle housing (102); and
characterised in that the catheter assembly (100) also comprises an elastic plug (124) capable of mating with said catheter hub (106), said plug (124) comprising a luer section insertable into the catheter hub (106) and a flange section (125) with a diameter no less than the diameter of the catheter hub (106) at its proximal end, whereby the needle (103) is insertable into the plug (124) by piercing through the flange section (125), which provides a positive stop for the distal end of the needle housing (102) having a blunt and flat nose (105).

2. The catheter assembly (100) of claim 1 wherein said catheter hub (106) contains a side port (122).

3. The catheter assembly (100) of claim 1 wherein said needle guard (107) further contains a push-off tab (116) on the opposite side of said needle guard guide.

4. The catheter assembly (100) of claim 1 wherein said needle guard guide is a longitudinal slot (111).

5. The catheter assembly (100) of claim 4 wherein the catheter (101) with needle guard (107) has a length greater than the length of the needle (103) and whose inner diameter is sufficient for it to surround all the elements of the catheter assembly (100) but for said gripping means (108).

6. The catheter assembly (100) of claim 4 wherein locking means (112) are provided on said slot (111) and said longitudinal guide means (109) locking the needle guard (107) in a position totally advanced with respect to the catheter (101).

7. The catheter assembly (100) according to claim 5, wherein the cylindrical central body of said needle housing (102) is provided with two transverse guiding ribs whose outer diameter is sized to fit the internal surface of said needle guard (107).

8. Catheter (101) according to claim 6 wherein said needle housing (102) is provided with four longitudinal guiding ribs as part of said cylindrical body such that said ribs fits within internal surface of said needle housing (102).

9. Catheter (101) according to claim 6 wherein said gripping means (108) has a partially interrupted cylindrical internal surface.

10. Catheter (101) according to claim 6 wherein said longitudinal guide means which connects the central body of said needle housing (102) to said gripping means (108), and which cooperates with said longitudinal slot (111) or guiding said needle guard (107), is made of two separate portions both tapered to a greater width towards the central portion of said guide means, and separated by an aperture in said central portion, and wherein locking means (112) are provided on said guide means consisting of a aperture in the central portion of said guide means, while the corresponding locking means on said needle guard (107) consist of two opposed teeth projecting along said guiding slot (111).

11. Catheter (101) according to claim 10 wherein said longitudinal slot (111) is provided with a pair of opposed teeth having a rounded profile and located towards the distal end of said needle guard (107).

12. Catheter (101) according to claim 6 wherein said cylindrical central body of said needle housing (102) is made of a transparent or translucent material.

13. Catheter (101) according to claim 6 also comprising a microporous plug closing the rear end of said central cylindrical portion.

## Patentansprüche

1. Katheterbaugruppe (100) bestehend aus:
einem an eine Katheterbuchse (106) angefügten Katheter (101);
einer Nadel (103), die zum Einsetzen in die Katheterbuchse (106) und in den Katheter (101) geeignet ist;
einem Nadelgehäuse (102) mit einem distalen Ende zum Halten der Nadel (103), einem zylindrischen Mittelteil, an welchem die Nadel (103) angefügt wird, einer Griffeinrichtung (108) zum Bewegen des Nadelgehäuses (102) und einer Längsführungseinrichtung (109), welche die Griffeinrichtung (108) mit dem Mittelteil verbindet;
einem zylindrischen Nadelschutz (107) mit einem Paar offener Enden, wobei der Nadelschutz (107) eine Führung aufweist, die in die Führungseinrichtung (109) des Nadelgehäuses (102) paßt;
**dadurch gekennzeichnet, daß** die Katheterbaugruppe (100) weiterhin einen elastischen Stopfen (124) aufweist, der in die Katheterbuchse (106) paßt, wobei der Stopfen (124) einen in die Katheterbuchse (106) einsetzbaren Einpaßquerschnitt sowie einen Flanschquerschnitt (125) aufweist, dessen Durchmesser nicht kleiner ist als derjenige der Katheterbuche (106) an ihrem proximalen Ende, wodurch die Nadel (103) in den Stopfen (124) einsetzbar ist, indem der Flanschquerschnitt (125) durchstochen wird, was einen zwangsläufigen Stopp für das distale Ende des Nadelgehäuses (102) mit einer abgerundeten und flachen Nase (105) bildet.

2. Katheterbaugruppe (100) nach Anspruch 1, bei welcher die Katheterbuchse (106) einen Seitendurchlaß (122) aufweist.

3. Katheterbaugruppe (100) nach Anspruch 1, bei welcher der Nadelschutz (107) auf der zur Nadelschutzführung gegenüberliegenden Seite weiterhin einen Abdrückansatz (116) aufweist.

4. Katheterbaugruppe (100) nach Anspruch 1, bei welcher der Nadelschutz ein Längsschlitz (111) ist.

5. Katheterbaugruppe (100) nach Anspruch 4, bei welcher der Katheter (101) mit dem Nadelschutz (107) eine größere Länge als die Nadel (103) hat und sein Innendurchmesser ausreicht, um alle Elemente der Katheterbaugruppe (100) außer denjenigen der Griffeinrichtung (108) zu umschließen.

6. Katheterbaugruppe (100) nach Anspruch 4, bei welcher eine Rasteinrichtung (112) an dem Schlitz (111) und der Längsführungseinrichtung (109) vorgesehen ist, um den Nadelschutz in einer in bezug auf den Katheter (101) vollständig vorgeschobenen Position einzurasten.

7. Katheterbaugruppe (100) nach Anspruch 5, bei welcher das zylindrische Mittelteil des Nadelgehäuses (102) mit zwei Querführungsrippen versehen ist, deren Außendurchmesser so bemessen sind, daß sie an die Innenfläche des Nadelschutzes (107) angepaßt sind.

8. Katheter (101) nach Anspruch 6, bei welchem das Nadelgehäuse (102) mit vier Längsführungsrippen als Teil des zylindrischen Körpers versehen ist, so daß sich diese Rippen der Innenfläche des Nadelgehäuses (102) anpassen.

9. Katheter (101) nach Anspruch 6, bei welchem die Griffeinrichtung (108) eine teilweise unterbrochene zylindrische Innenfläche aufweist.

10. Katheter (101) nach Anspruch 6, bei welchem die Längsführungseinrichtung, welche das Mittelteil des Nadelgehäuses (102) mit der Griffeinrichtung (108) verbindet und welche mit dem Längsschlitz (111) zusammenwirkt, um den Nadelschutz (107) zu führen, aus zwei getrennten Teilen hergestellt ist, die beide derart abgeschrägt sind, daß die größere Breite dem Mittelabschnitt der Führungseinrichtung zugewandt und durch eine Öffnung im Mittelabschnitt unterbrochen ist, wobei auf der Führungseinrichtung eine Rasteinrichtung (112) vorgesehen ist, die aus einer Öffnung im Mittelabschnitt der Führungseinrichtung besteht, während die entsprechende Rasteinrichtung auf dem Nadelschutz (107) aus zwei einander gegenüberliegenden Zähnen besteht, die entlang des Führungsschlitzes (111) herausragen.

11. Katheter (101) nach Anspruch 10, bei welchem der Längsschlitz (111) mit einem Paar einander gegenüberliegender Zähne versehen ist, die ein abgerundetes Profil haben und zum distalen Ende des Nadelschutzes (107) hin angeordnet sind.

12. Katheter (101) nach Anspruch 6, bei welchem das zylindrische Mittelteil des Nadelgehäuses (102) aus durchsichtigem oder durchscheinendem Material hergestellt ist.

13. Katheter (101) nach Anspruch 6, welcher auch einen mikroporösen Stopfen aufweist, der das hintere Ende des zylindrischen Mittelabschnittes verschließt.

## Revendications

1. Cathéter équipé (100) comportant:
un cathéter (101) attaché à un embout de cathéter (36);
une aiguille (103) pouvant être insérée dans ledit embout de cathéter (106) et dans ledit cathéter (101);
une poignée-enveloppe d'aiguille (102) présentant une extrémité avant pour tenir ladite aiguille (103), un corps cylindrique central auquel ladite aiguille (103) est attachée, des moyens de prise (108) pour déplacer ladite poignée-enveloppe (102) de l'aiguille et des moyens de guidage longitudinal (109) reliant lesdits moyens de prise (108) audit corps central;
un protecteur cylindrique d'aiguille (107) avec une paire d'extrémités ouvertes, ledit protecteur (107) ayant un guide pouvant correspondre auxdits moyens de guidage (109) de ladite poignéeenveloppe (102) de l'aiguille; et
caractérisé par le fait que ledit cathéter équipe (100) comporte également un bouchon élastique (124) pouvant correspondre audit embout de cathéter (106), ledit bouchon (124) comprenant une portion en cône Luer qui peut s'insérer dans l'embout de cathéter (106) et, à son extrémité arrière, une portion formant flasque (125) d'un diamètre non inférieur au diamètre de l'embout de cathéter (106), ce par quoi, on peut insérer l'aiguille (103) dans le bouchon (124) en perçant la portion formant flasque (125), ce qui donne une butée positive pour l'extrémité avant de la poignée-enveloppe (102) de l'aiguille qui possède un nez (105) aplati et plat.

2. Cathéter équipé (100) de la revendication 1 dans lequel, ledit embout de cathéter (106) contient un orifice latéral (122).

3. Cathéter équipé (100) de la revendication 1 dans lequel, ledit protecteur d'aiguille (107) contient en outre, du côté opposé audit guide de protecteur d'aiguille, une oreille à pousser (116).

4. Cathéter équipé (100) de la revendication 1 dans lequel, ledit guide de protecteur d'aiguille est une fente longitudinale (111).

5. Cathéter équipé (100) de la revendication 4 dans lequel, avec le protecteur d'aiguille (107), le cathéter (101) a une longueur supérieure à la longueur de l'aiguille (103), protecteur dont le diamètre intérieur est suffisant pour qu'il entoure tous les éléments du cathéter équipé (100) à l'exception desdits moyens de prise (108).

6. Cathéter équipé (100) de la revendication 4 dans lequel des moyens de verrouillage (112) sont prévus sur ladite fente (111) et sur lesdits moyens de guidage longitudinal (109) pour verrouiller le protecteur d'aiguille (107) en une position totalement avancée par rapport au cathéter (101).

7. Cathéter équipé (100) selon la revendication 5 dans lequel le corps cylindrique central de ladite poignée-enveloppe (102) de l'aiguille est muni de deux nervures de guidage transversal dont le diamètre extérieur est dimensionné pour s'ajuster à la surface intérieure dudit protecteur d'aiguille (107).

8. Cathéter équipé (101) selon la revendication 6 dans lequel ladite poignée-enveloppe (102) de l'aiguille est munie de quatre nervures de guidage longitudinal, faisant partie dudit corps cylindrique de façon que lesdites rainures s'ajustent à l'intérieur de la surface intérieure de ladite poignée-enveloppe (102) de l'aiguille.

9. Cathéter équipé (101) selon la revendication 6 dans lequel, lesdits moyens de prise (108) ont une surface cylindrique intérieure partiellement interrompue.

10. Cathéter équipé (101) selon la revendication 6, dans lequel lesdits moyens de guidage longitudinal qui relient le corps central de ladite poignée-enveloppe (102) de l'aiguille auxdits moyens de prise (108) et qui collaborent avec ladite fente longitudinale (111) pour guider ledit protecteur d'aiguille (107) sont constitués de deux portions distinctes allant l'une et l'autre en s'élargissant en direction de la portion centrale desdits moyens de guidage et séparées par une ouverture dans ladite portion centrale, et dans lequel, sur lesdits moyens de guidage, sont prévus des moyens de verrouillage (112) consistant en une ouverture dans la portion centrale desdits moyens de guidage, tandis que les moyens de verrouillage correspondants sur ledit protecteur d'aiguille (107) consistent en deux dents opposées saillant le long de ladite fente de guidage (111).

11. Cathéter équipé (101) selon la revendication 10, dans lequel ladite fente longitudinale (111) est munie d'une paire de dents opposées à profil arrondi situé en direction de l'extrémité avant dudit protecteur d'aiguille (107).

12. Cathéter équipé (101) selon la revendication 6, dans lequel ledit corps cylindrique central de ladite poignée-enveloppe (102) de l'aiguille est fait d'un matériau transparent ou translucide.

13. Cathéter équipé (101) selon la revendication 6, comportant également un bouchon microporeux obturant l'extrémité arrière de ladite portion centrale cylindrique.
